(19) **Europäisches Patentamt / European Patent Office / Office européen des brevets**

(11) **EP 3 974 787 B1**

(12) **EUROPEAN PATENT SPECIFICATION**

(45) Date of publication and mention
of the grant of the patent:
**26.11.2025 Bulletin 2025/48**

(21) Application number: **20810422.4**

(22) Date of filing: **18.05.2020**

(51) International Patent Classification (IPC):
**G01G 19/50** *(2006.01)*   **A61B 5/0537** *(2021.01)*
**G16H 40/63** *(2018.01)*   **G16H 50/70** *(2018.01)*
**A61B 5/00** *(2006.01)*

(52) Cooperative Patent Classification (CPC):
**G01G 19/50; A61B 5/0537; A61B 5/4872;**
**G16H 40/63; G16H 50/70**

(86) International application number:
**PCT/JP2020/019579**

(87) International publication number:
**WO 2020/235517 (26.11.2020 Gazette 2020/48)**

(54) **BODY COMPOSITION MEASUREMENT SYSTEM, BODY COMPOSITION MEASUREMENT PROGRAM, AND COMPUTER-READABLE NON-TRANSITORY STORAGE MEDIUM**

SYSTEM ZUR MESSUNG DER KÖRPERZUSAMMENSETZUNG, PROGRAMM ZUR MESSUNG DER KÖRPERZUSAMMENSETZUNG UND COMPUTERLESBARES NICHTTRANSITORISCHES SPEICHERMEDIUM

SYSTÈME DE MESURE DE LA COMPOSITION CORPORELLE, PROGRAMME DE MESURE DE LA COMPOSITION  CORPORELLE ET SUPPORT DE MÉMOIRE NON TRANSITOIRE LISIBLE PAR ORDINATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.05.2019 JP 2019095199**

(43) Date of publication of application:
**30.03.2022 Bulletin 2022/13**

(73) Proprietor: **Tanita Corporation
Tokyo 174-8630 (JP)**

(72) Inventors:
• **KODAMA Miyuki
Tokyo 174-8630 (JP)**

• **KUMEKAWA Mayumi
Tokyo 174-8630 (JP)**
• **MUTO Yugo
Tokyo 174-8630 (JP)**

(74) Representative: **Eisenführ Speiser
Patentanwälte Rechtsanwälte PartGmbB
Postfach 10 60 78
28060 Bremen (DE)**

(56) References cited:
**EP-A1- 1 522 259      JP-A- 2001 321 350
JP-A- 2004 337 578      JP-A- 2004 337 578
JP-A- 2007 313 019      JP-A- 2008 006 222**

**Description**

CROSS-REFERENCE TO RELATED APPLICAITONS

This application claims the benefit of Patent Application No. 2019-095199 filed in Japan on May 21, 2019.

TECHNICAL FIELD

The present disclosure relates to a body composition measurement system, a body composition measurement program, and a computer-readable non-transitory storage medium for obtaining positioning information of measured values based on acquired measurements of body composition.

BACKGOUND TECNOLOGY

A body type determining apparatus according to EP 1 522 259 A1 comprises: an input unit, an impedance measuring unit, a calculation unit, a standard setting unit, and a body type determining unit, wherein the input unit inputs personal physical data; the impedance measuring unit measures a bioelectrical impedance; the calculation unit calculates a body mass index and a body composition index based on the personal physical data and the bioelectrical impedance; the standard setting unit sets a normal value obtained from a regression formula based on measured data of the body mass index and the body composition index, as a body type determination standard, and the body type determining unit determines a body type based on the body type determining standard; thereby making it possible to determine a body type in numeric values with good accuracy.

In order to provide a device, method and program for estimating a health index capable of reducing an error resulting from the data which becomes the base of an estimating equation, it is proposed in JP 2004 337578 A that a storage means stores a health index estimating equation in which the health index is expressed as a function of a human body composition data. A calculating means calculates the health index as an estimated value using the human body composition data inputted from an input means and the health index estimating equation stored in the storage means. When the health index is inputted from the input means together with the human body composition data, the health index estimating equation stored in the storage means is corrected using the inputted health index and the human body composition data, and the corrected health index estimating equation is stored in the storage means. Afterward, when the human body composition data is inputted from the input means, the calculation of the health index is executed using the corrected health index estimating equation.

Conventionally, body composition analyzers are known to obtain measured values of body composition based on information such as height, weight, age, and gender, and bioelectrical impedance of each part of the human body obtained by measurement.

From these acquired measurements of body composition, changes in the body composition of the subject can be known. For example, JP2004-041811A discloses a biometric device that compares a user's latest inputted basal metabolic rate with an already stored basal metabolic rate. In addition, JP2007-244728A discloses a body composition analyzer that compares the measured value of a user with a standard value, and calculates an evaluation of whether this user's measured value is low, high, or at a standard level, etc.

SUMMARY OF THE INVENTION

According to the body composition analyzer of JP2007-244728A, the user could only know whether the measured value of his/her body composition was higher or lower than the standard value, and could not obtain more detailed information about the position of his/her body composition in the population. In addition, it was unclear which population's measurements were used as the basis for evaluating the measurements.

One of the purposes of the present disclosure is to provide a body composition measurement system and a body composition measurement program by which a user can obtain information about the position of his/her own body composition in a population. It is also one of the objects of the present disclosure to provide a body composition measurement system and a body composition measurement program that can obtain an evaluation of the measured values of one's own body composition in comparison with a specific population.

MEANS FOR RESOLVING THE PROBLEM

A body composition measurement system of an embodiment is defined in claim 1.

With this configuration, the positioning information of the measured value of body composition of the user is obtained, and the positioning information can be used to determine the positioning of the measured values of the body composition of the user in the population. The positioning information is, for example, the deviation value. With this configuration, the user can obtain an evaluation of the measured values of his/her own body composition in comparison with a specific population. The population to be selected may be a population to which the user himself or herself belongs, or a population to which the user himself or herself does not belong.

The body composition measurement section may obtain the measured value by measuring the body composition of the user.

With this configuration, the body composition measurement section can obtain the measured values by measuring the body composition of the user.

The positioning information determination section may determine the positioning information of the measured value of the user by using a positioning calculation formula or a positioning table that specifies the relationship between the measured values of the body composition and the positioning information.

With this configuration, the positioning information of the measured values of the body composition of the user can be easily obtained using the positioning calculation formula or positioning table. The positioning calculation formula or positioning table is, for example, a deviation value calculation formula or a deviation value table.

The positioning information determination section may determine the positioning information based on the measured value of body composition and a population comprising a plurality of measured values of body composition.

With this configuration, positioning information in the population of the measured values of the body composition of users can be determined without using a positioning calculation formula or a positioning table.

The positioning formula or the positioning table may be generated from a population comprising a plurality of measured values of body composition.

With this configuration, the positioning calculation formula or the positioning table can be generated from a population comprising a plurality of measured values of body composition. The positioning formula or positioning table may be generated by a terminal device such as a body composition analyzer, a tablet computer, a smartphone, or the like, or may be generated by a server computer.

The positioning calculation formula or the positioning table may be prepared for each population of different categories, and the positioning information determination section may determine the positioning information using the positioning calculation formula or the positioning table derived from the population in the selected category.

With this configuration, the positioning of the measured values of the body composition of the user in the population of a specific category can be determined.

The body composition measurement system may further comprise a server for storing the population.

With this configuration, the population can be stored in the server.

The server may generate a positioning calculation formula or a positioning table from the population, and the positioning information determination section may obtain the positioning calculation formula or the positioning table from the server and stores it, and determines the positioning information of the measured values of the user using the stored positioning calculation formula or the positioning table.

With this configuration, since the positioning formula or the positioning table for determining the position information is generated by the server, when the server has collected new measured values to update the population and update the positioning formula or the positioning table, the positioning information determination section can obtain the new positioning formula or the positioning table from the serve, update the stored positioning formula and the positioning table, and determine the positioning information of the measured value of body composition of the user using the updated positioning formula and the positioning table. In particular, when the server obtains the data of the measured values of body composition of a new population, the positioning information for such new population can be obtained.

The positioning information determination section may select a population of a category to which the user belongs.

With this configuration, the user can obtain the positioning of the measured value of his/her own body composition in comparison with others who are assumed to be related to him/her.

The positioning information acquisition section may select the population of an arbitrary category selected by the user.

With this configuration, the user can obtain the positioning information of the measured value of his/her own body composition in an arbitrary category regardless of the attributes of the user. For example, even if the user is an ordinary person, he/she can obtain his/her positioning information in a population of professional athletes.

The measured value obtained by the body composition measurement section may be added to the population in the server.

With this configuration, the population can be easily updated as new measured values are added to the population by obtaining measured value of body composition in order to determine the positioning information.

A body composition measurement program of an embodiment is defined in claim 12.

With this configuration also, the positioning information of the measured value of body composition of the user is obtained, and the positioning information can be used to determine the positioning of the measured values of the body composition of the user in the population.

With this configuration also, the user can obtain an evaluation of the measured values of his/her own body composition in comparison with a specific population.

BRIEF DISCRIPTION OF THE DRAWINGS

Fig. 1 shows a block diagram of a configuration of a body composition measurement system in an embodiment;
Fig. 3 shows a block diagram of a configuration of the body composition analyzer in the embodiment;
Fig. 4 shows an example of a graph in the embodiment;
Fig. 5 shows an example of a deviation table in the embodiment; and
Fig. 6 shows a display screen of the measurement results in the embodiment.

DISCRIPTION OF THE EMBODIMENTS

The following is a description of the embodiments of the present disclosure with reference to the drawings. The form of implementation described below shows an example of implementing the present disclosure, and does not limit the present disclosure to the specific configuration described below. In implementing the present disclosure, specific configurations may be adopted as appropriate according to the form of implementation.

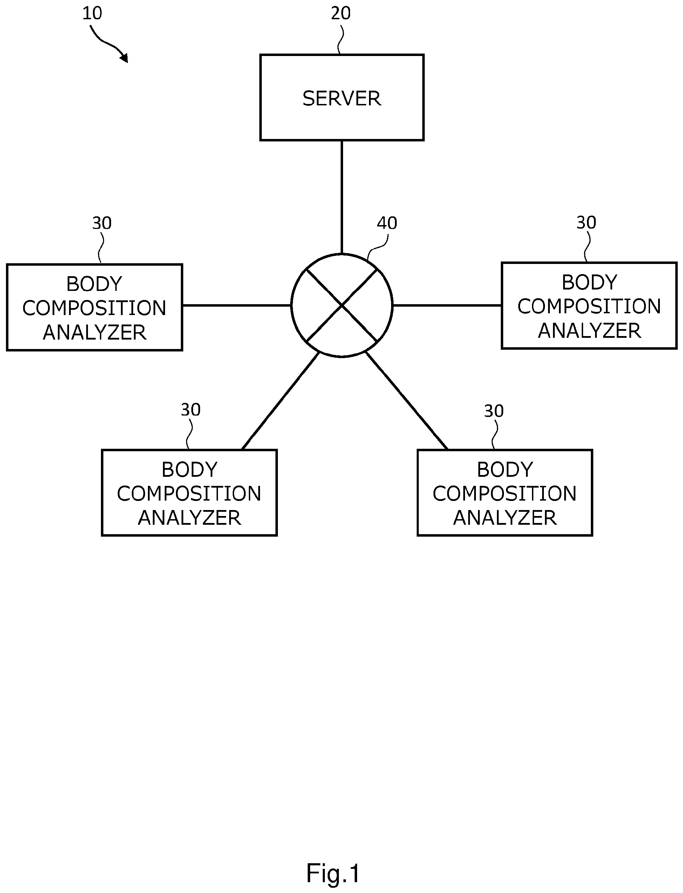

Fig. 1 shows a block diagram of a configuration of the body composition measurement system 10 in the embodiment. The body composition measurement system 10 is provided with a plurality of body composition analyzers 30 and a server 20 that is communicatively connected to the plurality of body composition analyzers 30 via a communication network 40. The communication network 40 may be a network closed within a predetermined organization, such as an intranet, or may be the Internet. The communication between the server 20 and each body composition analyzer 30 may be wired communication or wireless communication, and wireless communication may be partially used.

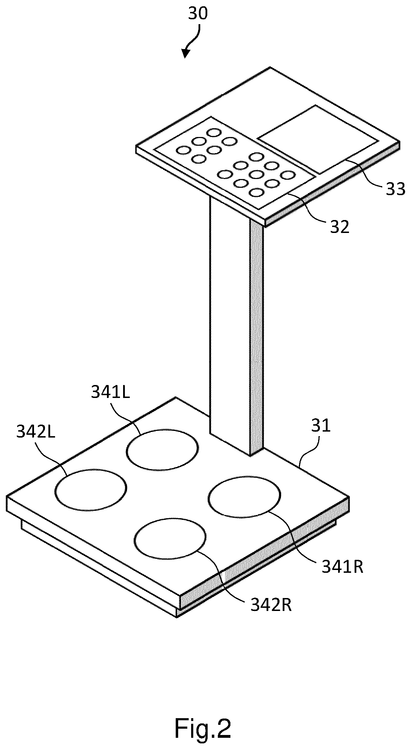

Fig. 2 shows a body composition analyzer 30 in the embodiment. The body composition analyzer 30 can measure body weight and body composition as biometric information. The body composition analyzer 30 is equipped with a main unit 31, an input unit 32, and an output unit 33.

The main unit 31 is equipped inside with a load cell for measuring weight, and can measure the weight of a user. The main unit 31 has an electrode 341L for current flow and an electrode 342L for measurement on the left side of the top surface, and an electrode 341R for current flow and an electrode 342R for measurement on the right side of the top surface. The user stands upright with bare feet on top of the main unit 31 to measure biometric data. At this time, the base of the toes of the left foot contacts the electrode 341L for current flow, and the heel of the left foot contacts the electrode 342L for measurement. The base of the toes of the right foot contacts the electrode 341R for current flow, and the heel of the right foot contacts the electrode 342R for measurement.

The body composition analyzer 30 is, for example, a four-electrode body composition analyzer that measures bioelectrical impedance by flowing current through the electrodes 341L and 341R for current flow and measuring the potential difference at the electrodes 342L and 342R for measurement. When the body composition analyzer 30 is an eight-electrode type, the body composition analyzer 30 can also measure the bioelectrical impedance of each part of the body.

The input unit 32 is used to input information into the body composition analyzer 30. The method of inputting information by the input unit 32 may be, for example, a manual method, a method via a recording medium, a method via wired communication, a method via wireless communication, or any other method.

The manual input method may be, for example, a button type, a dial type, or a touch sensor type. The recording medium of the method via a recording medium may be, for example, flash memory, CD-ROM, or DVD-ROM. The wireless communication of the method via wireless communication may be, for example, the Internet, a wireless LAN such as Wi-Fi (registered trademark), Bluetooth (registered trademark), NFC (Near Field Communication), or other short-range wireless communication.

The user operates the input unit 32 to input the user's information into the body composition analyzer 30. The user may, for example, input the measured values of the body composition obtained by a measurement device outside the body composition measurement system 10. The user may also input, for example, information such as the user's height, age, and gender, and the body composition analyzer 30 may obtain the measured values of the body composition by combining such information with the weight obtained in the measurement, the bioelectrical impedance, and the like. The body composition analyzer 30 measures, for example, the body fat percentage, body fat mass, muscle mass, abdominal/back muscle ratio, body water content, bone mass, visceral fat, and basal metabolism as the measured values of body composition. The measured values of body composition to be input do not have to be actual measured values. For example, fictitious measured values of body composition that the user is interested in may be input.

The output unit 33 is used to output the measurement results of the body composition analyzer 30. The output unit 33 is, for example, a display panel equipped with an LCD (Liquid Crystal Display) or an OLED (Organic Light Emitting Diode). The output unit 33 outputs, for example, measurement results such as body weight and body composition measurements. The output unit 33 may, for example, output numerical values, text, sound, or other formats that reflect the measurement results of the user.

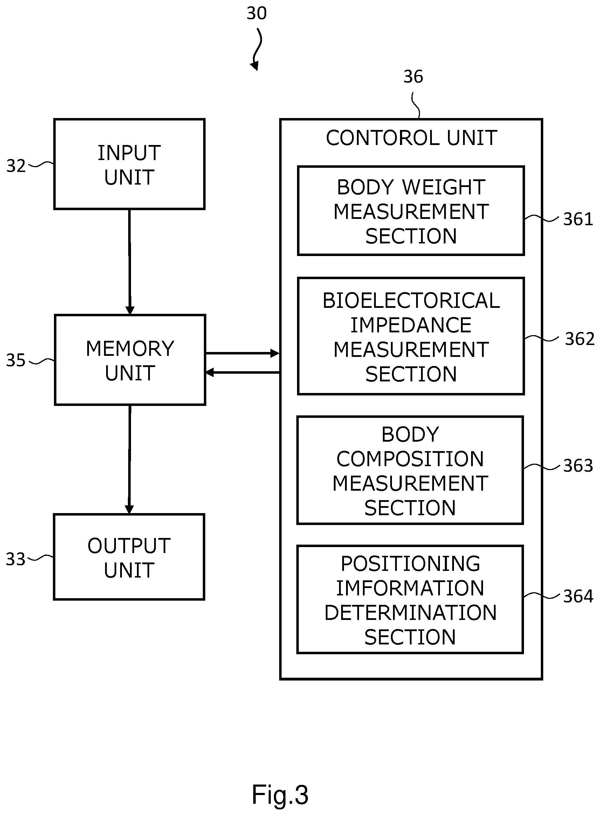

Fig. 3 shows a block diagram of a configuration of the body composition analyzer in the embodiment. The body composition analyzer 30 has an input unit 32, a memory unit 35, an output unit 33, and a control unit 36.

The memory unit 35 is a memory. The memory may be, for example, a volatile memory (e.g., RAM (Random Access Memory)), a non-volatile memory (e.g., ROM (Read Only Memory)), or the like. The memory unit 35 stores, for example, a program to be executed by the control unit 36, information input by a user by operating the input unit 32, statistical information for the body composition analyzer 30 to obtain measured values of body composition, measured values of body composition obtained by the body composition analyzer 30, and the like. In addition, the memory unit 35 stores, for example, a positioning calculation formula or a positioning table to be described later for determining positioning information of the measured values of the user.

The control unit 36 is a control device that controls the input unit 32, the memory unit 35, the output unit 33, the weight measuring section 361, the bioelectrical impedance measuring section 362, the body composition measuring section 363, and the positioning information determination section 364. The control unit 36 is equipped with a central processing section (CPU). The control unit 36 is connected to each section and controls the operation of each section. The control unit 36 realizes the functions of each part by executing the body composition measurement program of the present embodiment stored in the memory unit 35. The functions of each part may be realized by individual hardware such as an ASIC (Application Specific Integrated Circuit). The body composition measurement program may be provided to the body composition analyzer 30 by downloading it from a communication network, or may be provided to the body composition analyzer 30 via a non-transitory recording medium.

The weight measuring section 361 measures the weight of the user. The weight measuring section 361 measures the weight using the load cell described above. Specifically, the load cell consists of a straining body of a metal member that deforms in response to a load, and a strain gauge affixed to the straining body. When a user rides on top of the body composition analyzer 30, the load of the user causes the load cell's straining body to bend and the strain gauge to expand and contract. The resistance value (output value) of the strain gauge changes in accordance with the expansion and contraction. The weight measuring section 361 calculates the weight from the difference between the output value of the load cell when no load is applied (zero point) and the output value when a load is applied. The same configuration as in general scales can be used for the measurement of weight using the load cell.

The bioelectrical impedance measurement section 362 obtains the value of the bioelectrical impedance by measurement. The bioelectrical impedance measurement section 362 obtains the value of bioelectrical impedance by passing a weak current through the body via the electrodes 341L and 341R for current flow and the electrodes 342L and 342R for measurement shown in Fig. 2.

The body composition measurement section 363 obtains the measured values of the body composition. The measured values of the body composition may be obtained, for example, by inputting the measured values of the body composition obtained by a measurement device outside the body composition measurement system 10 as described above. The measured values of body composition may be obtained, for example, based on the bioelectrical impedance method as described above, based on the obtained bioelectrical impedance values and information such as height, age, gender, and weight. The body composition measurement section 363 may be provided in the body composition analyzer 30.

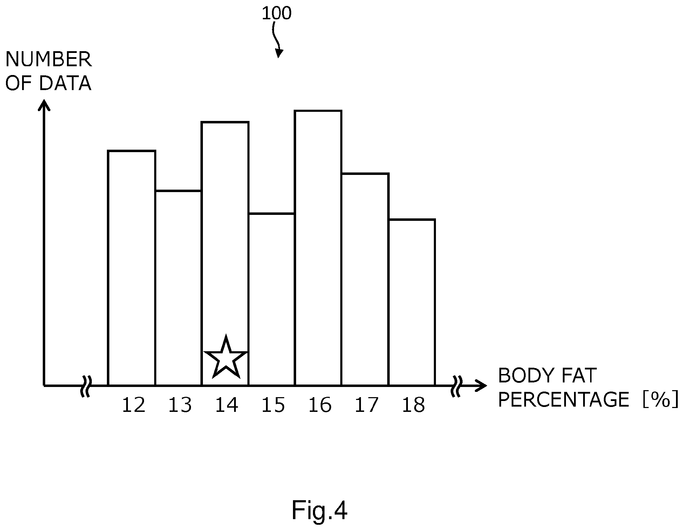

The positioning information determination section 364 determines positioning information of the measured values using the obtained measured values of body composition. The positioning information is information indicating the position in the population. The positioning information is superior to the classification into three values, such as higher or lower than the standard value, in that the positioning can be known in a concrete manner. In this embodiment, the positioning information is a deviation value. The positioning information may be generated, for example, by indicating the position to which one's own body composition data belongs (star) in a graph 100 showing the distribution of body composition data in an arbitrary population as shown in Fig. 4, or by indicating the rank in the population, probability of existence, etc. The population may be an imaginary population or an ideal population. The positioning information determination section 364 may be provided in any of the terminal devices such as the server 20, the body composition analyzer 30, the tablet computer, the smartphone, and the like. In the present embodiment, the positioning information determination section 364 is provided in the body composition analyzer 30.

The memory unit 35 stores a positioning calculation formula or a positioning table that is used by the positioning information determination section 364 to determine the positioning information. The positioning calculation formula is a formula for calculating the positioning information of measured values by substituting the measured values for which the positioning information is to be determined. The positioning table is a table that defines the positioning information for each measured value. When a measured value for which positioning information is to be obtained is obtained, the positioning information corresponding to this measured value can be obtained by referring to the positioning table. In this embodiment, the positioning calculation formula or positioning table is a deviation value calculation formula or deviation value table. The deviation calculation formula includes the mean value and standard deviation of the measured values of a specific population as coefficients.

The body composition analyzer 30 downloads the deviation value calculation formula or deviation value table from the server 20 via the communication network 40 and stores it in the memory unit 35. The server 20 generates the deviation value calculation formula or deviation value table. The server 20 obtains the measured values of the plurality of body compositions that serve as the population for generating the deviation value calculation formula or deviation value table.

As described above, since the measured values of body composition include a plurality of items such as body fat percentage, body fat mass, muscle mass, etc., the server 20 calculates a deviation value calculation formula or a deviation value table for each item of the measured values of body composition. In addition to calculating the deviation value calculation formula or deviation value table for the existing items, the server 20 may also calculate the deviation value calculation formula or deviation value table for the items obtained by combining the existing items. For example, an evaluation value for the item "difficulty in gaining weight" may be obtained by combining body fat percentage, muscle mass, and basal metabolic rate, and a deviation value calculation formula or deviation value table may be calculated for this evaluation value.

As is well known, the deviation value is obtained by the deviation value calculation formula (1).

$$T_i = 10 \times \frac{x_i - \bar{x}}{s} + 50 \quad \cdots (1)$$

Where, $T_i$ is the individual deviation value, $x_i$ is the individual measured value of body composition, x bar is the mean value of the measured values of body composition in a given population, and s is the standard deviation of the measured values of body composition in a given population.

The standard deviation s is obtained by the following formula (2).

$$s = \sqrt{\frac{1}{n} \sum_{n=1}^{n} (x_i - \bar{x})^2} \quad \cdots (2)$$

Where, n is the total number of data (measured values of body composition).

The server 20 prepares a population of measured values by dividing the plurality of obtained measured values into categories such as, for example, age, gender, region, country, race, occupation, sport type, company, etc. of users, and calculates the standard deviation for each of these populations using formula (2) to generate the deviation calculation formula (1).

At this time, one person's data (measured values of body composition) may belong to multiple populations. The populations may be prepared for different periods of time, for example, seasons, specific periods from the past to the present, etc. The input unit 32 of the body composition analyzer 30 downloads the deviation value calculation formula generated by the server 20 from the server 20 via the communication network 40 and stores it in the memory unit 35.

The server 20 may create a deviation value table from the deviation value calculation formula and provide the deviation value table to the body composition analyzer 30 in place of or in addition to the deviation value calculation formula described above. In this case, the body composition analyzer 30 downloads the deviation value table from the server 20 and stores it in the memory unit 35. The deviation value table specifies the relationship between the measured values and the deviation values in a tabular form based on the generated deviation value calculation formula.

The server 20 may also calculate the relationship between the measured values and the probability of existence in advance and include it in the deviation table to provide it to the body composition analyzer 30. The probability of existence can be obtained by calculating, for each measured value, the percentage of the population that the measured value falls within.

The server 20 can calculate the deviation value calculation formula or generate the deviation table that specifies the relationship between each measured value and the deviation value and the probability of existence in the same way for other body composition items such as body fat percentage, body fat mass, muscle mass, abdominal/back muscle ratio, body water content, bone mass, visceral fat, and basal metabolism described above, as well as for other categories such as age, gender, and region described above.

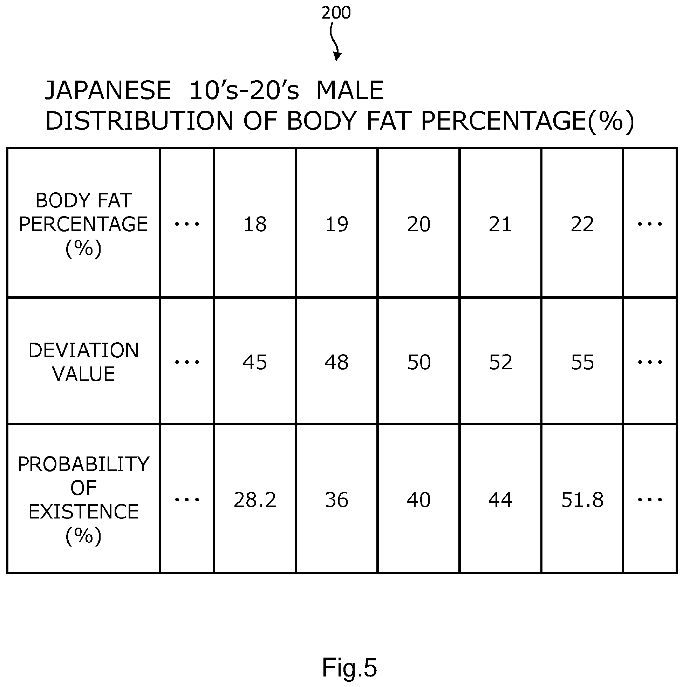

Fig. 5 shows an example of a deviation table 200 of an embodiment. The deviation table 200 maps the body fat percentage (%), the deviation value, and the probability of existence of "Japanese teens and twenties males." In the deviation table 200, integer values, which are discrete values, are specified as measured values.

In the case where the deviation value is obtained from the measured value using the deviation value table 200, when the body composition measuring section 363 obtains the body fat percentage as the measured value, to a decimal section, the positioning information determination section 364 refers to the deviation value table 200 by rounding off (or rounding down or rounding up) the decimal point.

For example, when the body fat percentage measured by the body composition measuring section 363 is 21.2%, the body composition analyzer 30 rounds it off to 21% body fat percentage and refers to the deviation value table 200 to obtain the deviation value 52 and the probability of existence 44%. Alternatively, in the deviation table 200, the measured values may be specified by ranges.

In the case where the positioning information determination section 364 obtains the deviation value calculation formula (1) from the server 20, the server 20 calculates the deviation value of this measurement by substituting the measured value of body composition measured by the body composition measurement section 363 into the deviation value calculation formula (1).

A plurality of categories of deviation value calculation formulas or deviation value tables are stored in the memory unit 35 for each item of measured values of body composition. The positioning information determination section 364 selects and uses one of these plural deviation value calculation formulas or deviation value tables to obtain the deviation value of the measured value measured by the body composition measurement section 363. In this case, which category of the deviation value calculation formula or the deviation value table is selected by the positioning information determination section 364 may be automatically determined by the positioning information determination section 364, or may be determined by the user by operating the input unit 32 to make a designation.

Specifically, in the case where the positioning information determination section 364 automatically selects a category, the positioning information determination section 364 may automatically select a category that matches the attribute information of the user. For example, if the positioning information determination section 364 knows, as an attribute of the user, that the user is an athlete of a specific sport, the positioning information determination section 364 may select a category whose population consists of the measured values of athletes of this sport. Also, for example, if the positioning information determination section 364 knows, as an attribute of the user, that the time of day for measurement is mostly at night, it may select a category whose population is the measured values of users of this measurement habit.

In the case where the user selects the category arbitrarily, the user can select the population regardless of his/her own attributes and obtain the deviation value. For example, an ordinary person who exercises every day to improve his or her health can select a category whose population is the measured values of players of a particular professional sports team and find out his or her own deviation value in the category. Also, by selecting a category whose population consists of the measured values of people whose occupation is different from your own, you can use it to determine whether or not your own physical strength is sufficient for the type of job you want to change. Furthermore, by devising ways to create categories, various ways of enjoying and using the system can be provided.

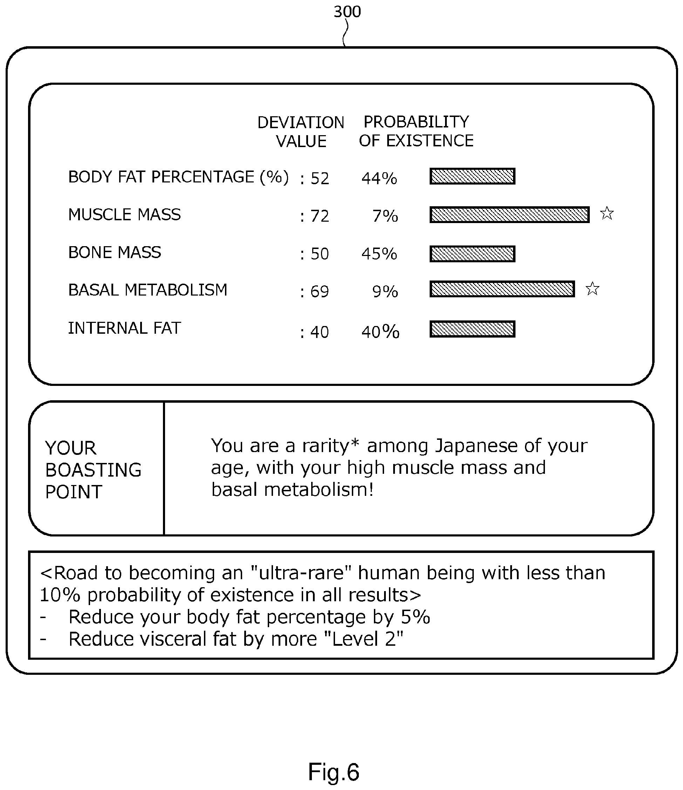

Fig. 6 shows the display screen 300 of the measurement results of the embodiment. This display screen 300 is displayed on the output unit 33. In the display screen 300, the deviation value of the measured value of each item of body composition and its probability of existence, "your boasting point" that explains the user's superiority in comparison with others, and the content that explains the amount of change in the measured value of body composition to achieve a deviation value and probability of existence superior to the current state are displayed.

Specifically, in the display screen 300 of the example shown in Fig. 6, the deviation values of the measured values and their probabilities of existence are displayed as follows: body fat percentage, probability of existence 44% with a deviation value of 52; muscle mass, probability of existence 7% with a deviation value of 72; bone mass, probability of existence 45% with a deviation value of 50; basal metabolism, probability of existence 9% with a deviation value of 69%; visceral fat, probability of existence 40% with a deviation value of 40. In particular, muscle mass and basal metabolism, which have low probability of existence, are marked with a star to distinguish them from other items.

In addition, as a "your boasting point", "You have a high muscle mass and high basal metabolism among Japanese people of the same age." will be displayed. "<Road to becoming an "ultra-rare" human being with less than 10% probability of existence in all results> / Reduce your body fat percentage by 5% / Reduce visceral fat by more "Level 2"" will be displayed. The target may be set by the user, or automatically based on the results of the current deviation, probability of existence, etc.

The measured values of the body composition obtained in the body composition measurement section 363 are used in the positioning information determination section 364 to determine the deviation values, and are also sent from the body composition analyzer 30 to the server 20 via the communication network 40. At this time, the body composition analyzer 30 sends the measured values of body composition to the server 20 together with the attributes of the user of the measured values of body composition.

The server 20 obtains the measured values of body composition and the attributes of the users from the plurality of body composition analyzers 30 and adds them to the population of the corresponding category. The server 20 adds new data to the population in this way. Periodically or when instructed to do so, the server 20 re-generates the deviation value calculation formula using the new population, and when generating the deviation value table, it re-generates the deviation value table using the new population.

The body composition analyzer 30 updates the deviation value calculation formula or the deviation value calculation table by downloading the deviation value calculation formula or the deviation value table newly generated by the server 20 and replacing the deviation value calculation formula or the deviation value table stored in the memory unit 35 with the new deviation value calculation formula or the deviation value table downloaded. The body composition analyzer 30 may be updated periodically, in response to instructions from the user, or in response to other triggers (e.g., when the body composition analyzer 30 is started).

As described above, in this embodiment, the body composition analyzer obtains the measured values of body composition based on the bioelectrical impedance method. Then, the body composition analyzer obtains the deviation value of the measured value based on this measured value and the deviation value calculation formula or deviation value table of the category to which the user belongs, and the user can obtain the position of the measured value of body composition in the population.

In addition, since the deviation value table and the deviation value calculation formula for obtaining deviation values are generated by the server 20, when the server 20 updates the population by collecting new measured values and updates the deviation value table or the deviation value calculation formula, the positioning information determination section 364 obtains the new deviation value table or the deviation value calculation formula from the server 20, updates the stored deviation value table or deviation value calculation formula, and determines the deviation value of the measured value of body composition of the user using the updated deviation value table or the updated deviation value calculation formula. In this case, the population can be easily updated because new measured values are added to the population by obtaining measured values of body composition to determine the deviation value for the user.

In addition, since the server 20 collects the body composition measurement values of multiple users to update the population, not only the change in one's own body composition but also the change in the body composition of other users becomes an element of the deviation value of one's own body composition. Therefore, even if there is no change in the body composition of one user, the deviation value of body composition of that user may change, which prevents the user from getting bored and is expected to improve health awareness.

In addition, since the deviation value calculation formula or the deviation value table is periodically updated and periodically downloaded to the body composition analyzer, the deviation values of the measured values can be determined based on the updated deviation value calculation formula or the deviation value table.

(Variant examples)

In the above-described embodiment, the main unit 31, the input unit 32, the output unit 33, the display unit 34, the memory unit 35, and the control unit 36 were integrated to constitute the body composition analyzer 30, but the components other than the main unit 31 can be provided in an information processing device different from the body composition analyzer 30, and the body composition analyzer 30 of the present embodiment can consist of such a body composition analyzer and the information processing device. In this case, the information processing device and the body composition analyzer 30 communicate with each other by wired or wireless means. The information processing device may be, for example, an information processing device such as a smartphone or a tablet computer.

The positioning information determination section 364 may be provided on the server 20 side instead of the body composition analyzer 30 side. In this case, the body composition analyzer 30 does not need to download the deviation value calculation formula and the deviation value table from the server 20, but transmits the measured values of the body composition obtained by the body composition measurement section 363 to the server 20 via the communication network 40, and the server 20 obtains the deviation values by the positioning information determination section 364 and returns them to the body composition analyzer 30. If the mean value x bar and the standard deviation s of a given population are stored in the server 20, the server 20 can obtain the deviation value from the mean value x bar and the standard deviation s and return it to the body composition analyzer 30 without being based on the population.

In the above-described embodiment, the server 20 generates a deviation value calculation formula and a deviation value table from the measured values of the plurality of body compositions that serve as the population and supplies them to each body composition analyzer 30, but the body composition analyzer 30 may have a function to generate the deviation value calculation formula and the deviation value table. In this case, a plurality of body composition measured value are provided to the body composition analyzer 30 from the server 20, and the body composition analyzer 30 generates a deviation value calculation formula and a deviation value table using the data of the population for each category. If the mean value x bar and the standard deviation s of a given population are stored in the memory unit 35 as statistical information, the positioning information determination section 364 can generate a deviation value calculation formula and a deviation value table from the mean value x bar and the standard deviation s without being based on the population.

In the above-described embodiment, when the body composition analyzer 30 obtains a new deviation value calculation formula or deviation value table from the server 20, the old deviation value calculation formula or deviation value table is updated by deleting the old formula or deviation value table and replacing it with the new deviation value calculation formula or deviation value table, but alternatively, the body composition analyzer 30 can maintain the old deviation value calculation formula or deviation value table without deleting the old deviation value calculation formula and deviation value table in the body composition analyzer 30, and

The measured values of the body composition obtained by the body composition measuring section 363 may be stored in the memory unit 35, and the positioning information determination section 364 may bring up the measured values of the body composition in the past and obtain the deviation value using the latest or any past deviation value calculation formula or deviation value table.

In the above-described body composition measurement system 10, the deviation value was employed to obtain an evaluation when the measured values of the body composition of the user are compared with the measured values of the body composition of a specific population, but the body composition measurement system 10 does not necessarily need to employ deviation values to obtain an evaluation of the measured body composition of the user compared to the measured body composition of various populations.

In other words, the body composition measurement system 10 of this embodiment is equipped with a body composition measurement section 363 that obtains measured values by measuring the body composition of a user, and an evaluation section that determines an evaluation of the measured values of the body composition of the user with respect to the body composition of a selected population among a plurality of populations having different categories. The evaluation of the measured values of the body composition of the user may be, for example, a result of comparison with an average of the measured values of the body composition of the selected population, or a probability of existence. The positioning information determination section 364 of the above-described system is one example of the evaluation section. With this body composition measurement system 10, it is possible to obtain an evaluation of one's own body composition measured values in comparison with a specific population.

[Explanation of signs]

10: Body composition measurement system
20: Server
30: Body composition analyzer
40: Communication network
31: Main unit
32: Input unit
33: Output unit
341L, R: Electrodes for current flow
342L, R: Electrodes for measurement
35: Memory unit
36: Control unit
361: Body weight measurement section
362: Bioelectrical impedance measurement section
363: Body composition measurement section
364: Positioning information determination section
100: Graph
200: Deviation table
300: Display screen

**Claims**

1. A body composition measurement system (10), comprising:

   a body composition measurement section (363) for obtaining a measured value of body composition of a user;

**characterized by**
an evaluation section (364) for determining positioning information or evaluation, wherein
- the positioning information is information indicating the position of the measured value of the user with respect to the body composition of a selected population among a plurality of populations whose categories are different from each other and which are formed from measured values and
- the evaluation is an evaluation of the measured value of the user with respect to the body composition of a selected population among a plurality of populations whose categories are different from each other and which are formed from measured values.

2. The body composition measurement system (10) according to claim 1, wherein the body composition measurement section (363) obtains the measured value by measuring the body composition of the user.

3. The body composition measurement system (10) according to claim 1 or 2, wherein the evaluation section (364) determines the positioning information or evaluation of the measured value of the user by using a positioning calculation formula or a positioning table (200) that specifies the relationship between the measured values of the body composition and the positioning information or evaluation.

4. The body composition measurement system (10) according to claim 1 or 2, wherein the evaluation section (364) determines the positioning information or evaluation based on the measured value of body composition and the selected population comprising a plurality of measured values of body composition.

5. The body composition measurement system (10) according to claim 3, wherein the positioning formula or the positioning table (200) is generated from a population comprising a plurality of measured values of body composition.

6. The body composition measurement system (10) according to claim 5, wherein the positioning calculation formula or the positioning table (200) is prepared for each population of the plurality of populations of different categories, and the evaluation section (364) determines the positioning information or evaluation using the positioning calculation formula or the positioning table (200) derived from the population in the selected category.

7. The body composition measurement system (10) according to any of claims 4 to 6, further comprising a server (20) for storing the population.

8. The body composition measurement system (10) according to claim 7, wherein the server (20) generates a positioning calculation formula or a positioning table (200) from the population, and
the evaluation section (364) obtains the positioning calculation formula or the positioning table (200) from the server (20) and stores it, and determines the positioning information or evaluation of the measured values of the user using the stored positioning calculation formula or the positioning table (200).

9. The body composition measurement system (10) according to any of claims 4, 6 and 8, wherein the evaluation section (364) selects a population of a category to which the user belongs.

10. The body composition measurement system (10) according to any of claims 4, 6, and 8, wherein the evaluation section (364) selects the population of a category selected by the user.

11. The body composition measurement system (10) according to claim 7 or 8, wherein the measured value obtained by the body composition measurement section (363) is added to the population in the server (20).

12. A body composition measurement program, that causes a computer (30) to function as:

a body composition measurement section (363) for obtaining a measured value of body composition of a user;
**characterized in that** the program further causes the computer (30) to function as
an evaluation section (364) for determining positioning information or evaluation, wherein
- the positioning information is information indicating the position of the measured value of the user with respect to the body composition of a selected population among a plurality of populations whose categories are different from each other and which are formed from measured values, and
- the evaluation is an evaluation of the measured value of the user with respect to the body composition of a selected population among a plurality of populations whose categories are different from each other and which are formed from measured values.

**13.** A computer-readable non-transitory storage medium storing a body composition measurement program according to claim 12.

**Patentansprüche**

1. Körperzusammensetzungsmesssystem (10), mit:

   einem Körperzusammensetzungsmessabschnitt (363) zum Erhalten eines Messwerts der Körperzusammensetzung eines Benutzers;
   **gekennzeichnet durch**
   einen Bewertungsabschnitt (364) zum Bestimmen von Positionierungsinformationen oder einer Bewertung, wobei
   - die Positionierungsinformationen Informationen sind, die die Position des Messwerts des Benutzers in Bezug auf die Körperzusammensetzung einer ausgewählten Population aus mehreren Populationen angeben, deren Kategorien sich voneinander unterscheiden und die aus Messwerten gebildet sind, und
   - die Bewertung eine Bewertung des Messwerts des Benutzers in Bezug auf die Körperzusammensetzung einer ausgewählten Population aus mehreren Populationen ist, deren Kategorien sich voneinander unterscheiden und die aus Messwerten gebildet sind.

2. Körperzusammensetzungsmesssystem (10) nach Anspruch 1, wobei der Körperzusammensetzungsmessabschnitt (363) den Messwert durch Messen der Körperzusammensetzung des Benutzers erhält.

3. Körperzusammensetzungsmesssystem (10) nach Anspruch 1 oder 2, wobei der Bewertungsabschnitt (364) die Positionierungsinformationen oder die Bewertung des Messwerts des Benutzers unter Verwendung einer Positionierungsberechnungsformel oder einer Positionierungstabelle (200) bestimmt, die die Beziehung zwischen den Messwerten der Körperzusammensetzung und den Positionierungsinformationen oder der Auswertung angibt.

4. Körperzusammensetzungsmesssystem (10) nach Anspruch 1 oder 2, wobei der Bewertungsabschnitt (364) die Positionierungsinformation oder die Bewertung auf Grundlage des Messwerts der Körperzusammensetzung und der ausgewählten Population, die mehrere Messwerte der Körperzusammensetzung umfasst, bestimmt.

5. Körperzusammensetzungsmesssystem (10) nach Anspruch 3, wobei die Positionierungsberechnungsformel oder die Positionierungstabelle (200) aus einer Population erzeugt wird, die mehrere Messwerte der Körperzusammensetzung umfasst.

6. Körperzusammensetzungsmesssystem (10) nach Anspruch 5, wobei die Positionierungsberechnungsformel oder die Positionierungstabelle (200) für jede Population der mehreren Populationen verschiedener Kategorien erstellt wird, und
   der Bewertungsabschnitt (364) die Positionierungsinformationen oder die Bewertung unter Verwendung der Positionierungsberechnungsformel oder der Positionierungstabelle (200) bestimmt, die aus der Population in der ausgewählten Kategorie abgeleitet wurde.

7. Körperzusammensetzungsmesssystem (10) nach einem der Ansprüche 4 bis 6, ferner mit einem Server (20) zum Speichern der Population.

8. Körperzusammensetzungsmesssystem (10) gemäß Anspruch 7, wobei der Server (20) eine Positionierungsberechnungsformel oder eine Positionierungstabelle (200) aus der Population generiert, und
   der Bewertungsabschnitt (364) die Positionsberechnungsformel oder die Positionierungstabelle (200) vom Server (20) abruft, sie speichert und die Positionierungsinformationen oder die Bewertung der Messwerte des Benutzers unter Verwendung der gespeicherten Positionsberechnungsformel oder der Positionierungstabelle (200) bestimmt.

9. Körperzusammensetzungsmesssystem (10) nach einem der Ansprüche 4, 6 und 8, wobei der Bewertungsabschnitt (364) eine Population einer Kategorie auswählt, zu der der Benutzer gehört.

10. Körperzusammensetzungsmesssystem (10) nach einem der Ansprüche 4, 6 und 8, wobei der Auswertungsabschnitt (364) die Population einer vom Benutzer ausgewählten Kategorie auswählt.

**11.** Körperzusammensetzungsmesssystem (10) gemäß Anspruch 7 oder 8, wobei der vom Körperzusammensetzungsmessabschnitt (363) erhaltene Messwert zur Population im Server (20) hinzugefügt wird.

**12.** Körperzusammensetzungsmessprogramm, das einen Computer (30) dazu veranlasst, als

ein Körperzusammensetzungsmessabschnitt (363) zum Erhalten eines Messwerts der Körperzusammensetzung eines Benutzers zu funktionieren;
**dadurch gekennzeichnet, dass** das Programm den Computer (30) ferner dazu veranlasst, als
ein Bewertungsabschnitt (364) zum Bestimmen von Positionierungsinformationen oder einer Auswertung zu funktionieren, wobei
- die Positionierungsinformationen Informationen sind, die die Position des Messwerts des Benutzers in Bezug auf die Körperzusammensetzung einer ausgewählten Population aus mehreren Populationen angeben, deren Kategorien sich voneinander unterscheiden und die aus Messwerten gebildet sind, und
- die Bewertung eine Bewertung des Messwerts des Benutzers in Bezug auf die Körperzusammensetzung einer ausgewählten Population aus mehreren Populationen ist, deren Kategorien sich voneinander unterscheiden und die aus Messwerten gebildet sind.

**13.** Computerlesbares, nichtflüchtiges Speichermedium, das ein Körperzusammensetzungsmessprogramm nach Anspruch 12 speichert.

**Revendications**

**1.** Système de mesure de composition corporelle (10), comprenant :

une section de mesure de composition corporelle (363) pour obtenir une valeur mesurée de composition corporelle d'un utilisateur ;
**caractérisé par**
une section d'évaluation (364) pour déterminer des informations de positionnement ou une évaluation, dans lequel
- les informations de positionnement sont des informations indiquant la position de la valeur mesurée de l'utilisateur par rapport à la composition corporelle d'une population sélectionnée parmi une pluralité de populations dont les catégories sont différentes les unes des autres et qui sont formées à partir de valeurs mesurées et
- l'évaluation est une évaluation de la valeur mesurée de l'utilisateur par rapport à la composition corporelle d'une population sélectionnée parmi une pluralité de populations dont les catégories sont différentes les unes des autres et qui sont formées à partir de valeurs mesurées.

**2.** Système de mesure de composition corporelle (10) selon la revendication 1, dans lequel la section de mesure de composition corporelle (363) obtient la valeur mesurée en mesurant la composition corporelle de l'utilisateur.

**3.** Système de mesure de composition corporelle (10) selon la revendication 1 ou 2, dans lequel la section d'évaluation (364) détermine les informations de positionnement ou l'évaluation de la valeur mesurée de l'utilisateur en utilisant une formule de calcul de positionnement ou une table de positionnement (200) qui spécifie la relation entre les valeurs mesurées de la composition corporelle et les informations de positionnement ou l'évaluation.

**4.** Système de mesure de composition corporelle (10) selon la revendication 1 ou 2, dans lequel la section d'évaluation (364) détermine les informations de positionnement ou l'évaluation sur la base de la valeur mesurée de composition corporelle et de la population sélectionnée comprenant une pluralité de valeurs mesurées de composition corporelle.

**5.** Système de mesure de composition corporelle (10) selon la revendication 3, dans lequel la formule de positionnement ou la table de positionnement (200) est générée à partir d'une population comprenant une pluralité de valeurs mesurées de composition corporelle.

**6.** Système de mesure de composition corporelle (10) selon la revendication 5, dans lequel la formule de calcul de positionnement ou la table de positionnement (200) est préparée pour chaque population de la pluralité de populations de différentes catégories, et
la section d'évaluation (364) détermine les informations de positionnement ou l'évaluation en utilisant la formule de calcul de positionnement ou la table de positionnement (200) dérivée de la population de la catégorie sélectionnée.

**7.** Système de mesure de composition corporelle (10) selon l'une quelconque des revendications 4 à 6, comprenant en outre un serveur (20) pour stocker la population.

**8.** Système de mesure de composition corporelle (10) selon la revendication 7, dans lequel le serveur (20) génère une formule de calcul de positionnement ou une table de positionnement (200) à partir de la population, et la section d'évaluation (364) obtient la formule de calcul de positionnement ou la table de positionnement (200) à partir du serveur (20) et la stocke, puis détermine les informations de positionnement ou l'évaluation des valeurs mesurées de l'utilisateur en utilisant la formule de calcul de positionnement ou la table de positionnement (200) stockée.

**9.** Système de mesure de composition corporelle (10) selon l'une quelconque des revendications 4, 6 et 8, dans lequel la section d'évaluation (364) sélectionne une population d'une catégorie à laquelle appartient l'utilisateur.

**10.** Système de mesure de composition corporelle (10) selon l'une quelconque des revendications 4, 6 et 8, dans lequel la section d'évaluation (364) sélectionne la population d'une catégorie sélectionnée par l'utilisateur.

**11.** Système de mesure de composition corporelle (10) selon la revendication 7 ou 8, dans lequel la valeur mesurée obtenue par la section de mesure de composition corporelle (363) est ajoutée à la population dans le serveur (20).

**12.** Programme de mesure de composition corporelle, qui amène un ordinateur (30) à fonctionner comme :

une section de mesure de composition corporelle (363) pour obtenir une valeur mesurée de composition corporelle d'un utilisateur ;
**caractérisé en ce que** le programme amène en outre l'ordinateur (30) à fonctionner comme une section d'évaluation (364) pour déterminer des informations de positionnement ou une évaluation, dans lequel
- les informations de positionnement sont des informations indiquant la position de la valeur mesurée de l'utilisateur par rapport à la composition corporelle d'une population sélectionnée parmi une pluralité de populations dont les catégories sont différentes les unes des autres et qui sont formées à partir de valeurs mesurées, et
- l'évaluation est une évaluation de la valeur mesurée de l'utilisateur par rapport à la composition corporelle d'une population sélectionnée parmi une pluralité de populations dont les catégories sont différentes les unes des autres et qui sont formées à partir de valeurs mesurées.

**13.** Support de stockage non transitoire lisible par ordinateur stockant un programme de mesure de composition corporelle selon la revendication 12.

Fig.1

Fig.2

30

36

INPUT UNIT

32

CONTOROL UNIT

BODY WEIGHT MEASUREMENT SECTION

361

BIOELECTORICAL IMPEDANCE MEASUREMENT SECTION

362

MEMORY UNIT

35

BODY COMPOSITION MEASUREMENT SECTION

363

OUTPUT UNIT

33

POSITIONING IMFORMATION DETERMINATION SECTION

364

Fig.3

Fig.4

200

## JAPANESE 10's-20's MALE
## DISTRIBUTION OF BODY FAT PERCENTAGE(%)

| BODY FAT PERCENTAGE (%) | ・・・ | 18 | 19 | 20 | 21 | 22 | ・・・ |
|---|---|---|---|---|---|---|---|
| DEVIATION VALUE | ・・・ | 45 | 48 | 50 | 52 | 55 | ・・・ |
| PROBABILITY OF EXISTENCE (%) | ・・・ | 28.2 | 36 | 40 | 44 | 51.8 | ・・・ |

Fig.5

300

|                            | DEVIATION VALUE | PROBABILITY OF EXISTENCE |        |   |
|----------------------------|-----------------|--------------------------|--------|---|
| BODY FAT PERCENTAGE (%)    | : 52            | 44%                      |        |   |
| MUSCLE MASS                | : 72            | 7%                       |        | ☆ |
| BONE MASS                  | : 50            | 45%                      |        |   |
| BASAL METABOLISM           | : 69            | 9%                       |        | ☆ |
| INTERNAL FAT               | : 40            | 40%                      |        |   |

| YOUR BOASTING POINT | You are a rarity* among Japanese of your age, with your high muscle mass and basal metabolism! |
|---|---|

<Road to becoming an "ultra-rare" human being with less than 10% probability of existence in all results>
- Reduce your body fat percentage by 5%
- Reduce visceral fat by more "Level 2"

Fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019095199 A
- EP 1522259 A1
- JP 2004337578 A
- JP 2004041811 A
- JP 2007244728 A